# EUROPÄISCHE PATENTSCHRIFT

(11) **EP 2 883 426 B1**
(45) Veröffentlichungstag und Bekanntmachung des Hinweises auf die Patenterteilung: **22.06.2016**
(21) Anmeldenummer: 13756295.5
(22) Anmeldetag: 18.07.2013
(51) Int. Cl.: H05H 1/24, A61N 5/00, A61B 18/00, A61N 1/00

(54) **ELEKTRODENANORDNUNG FÜR EIN BEHINDERTES PLASMA**
ELECTRODE ARRANGEMENT FOR A DIELECTRIC BARRIER DISCHARGE PLASMA
AGENCEMENT D'ÉLECTRODES POUR UN PLASMA À BARRIÈRE DIÉLECTRIQUE

(30) Priorität: 07.08.2012 DE 102012015482
(43) Veröffentlichungstag der Anmeldung: 17.06.2015
(73) Patentinhaber: Cinogy GmbH, 37115 Duderstadt (DE)
(72) Erfinder: WANDKE, Dirk, 37308 Heilbad Heiligenstadt (DE); HAHNL, Mirko, 37339 Berlingerode (DE); KOPP, Matthias, 37434 Gieboldehausen (DE); TRUTWIG, Leonhard, 37115 Duderstadt (DE); STORCK, Karl-Otto, 371115 Duderstadt (DE)
(74) Vertreter: Lins, Edgar
(86) Internationale Anmeldenummer: PCT/DE2013/000392
(87) Internationale Veröffentlichungsnummer: WO 2014/023276

(56) Entgegenhaltungen:
- WO-A1-2011/076193
- WO-A1-2012/065125
- WO-A2-2006/116252
- US-A1- 2011 313 417
- US-B1- 6 413 255

## Beschreibung

Die Erfindung betrifft eine Elektrodenanordnung zur Ausbildung eines dielektrisch behinderten Plasmas zwischen einer Wirkfläche der Elektrodenanordnung und einer als Gegenelektrode fungierenden Oberfläche, mit einer flexiblen, flächigen Elektrode, die mit einer Hochspannungsquelle verbindbar ist und mit einem flächigen, flexiblen und die Wirkfläche bildenden Dielektrikum, das mit der flächigen Elektrode zu einem Elektrodenelement verbunden ist und die Elektrode zur zu behandelnden Oberfläche hin vollständig abdeckt.

Es ist bekannt, dass durch Plasmaentladungen Oberflächen von Werkstücken, aber auch Hautoberflächen, unter verschiedenen Aspekten mit Vorteil behandelt werden können. Oberflächen von Werkstücken können für eine nachfolgende Oberflächenbehandlung, beispielsweise Beschichtung, aktiviert werden, um die Beschichtung besser haften zu lassen. Es ist ferner möglich, Oberflächen mit Plasmaentladungen zu reinigen, beispielsweise Ölschichten abzutragen.

Eine dielektrisch behinderte Plasmaentladung ermöglicht ferner die gefahrlose Behandlung von Hautoberflächen. Dadurch gelingt es beispielsweise Wundflächen schneller heilen zu lassen, die Hautoberfläche für verbesserte Aufnahme von pflegenden oder heilenden Wirkstoffen zu aktivieren usw.

Bei unregelmäßig dreidimensional geformten Oberflächen besteht das Problem, eine möglichst gleichmäßige Plasmabehandlung vornehmen zu können. Gemäß der DE 195 32 105 C2 ist es vorgesehen, von der Oberfläche des Werkstücks eine Negativform mit dem Dielektrikum auszubilden, das somit aus einem formbaren, beispielsweise pressbaren oder tiefziehbaren Kunststoff besteht. Vorgesehen ist dabei ferner, dass eine Zwischenschicht verwendet wird, sodass das Dielektrikum mit der Zwischenschicht unmittelbar an der Oberfläche des Werkstücks geformt werden kann. Die Zwischenschicht wird dann entfernt, um einen Zwischenraum zwischen dem Dielektrikum und der Elektrode zu gewährleisten, in dem sich das Plasma ausbilden kann. Das Dielektrikum wird auf seiner von der zu behandelnden Oberfläche abgewandten Seite mit einem leitenden Material beschichtet, das die Elektrode bildet und dem die benötigte hohe Spannung in Form einer Wechselspannung zuführbar ist.

Eine Elektrodenanordnung der eingangs erwähnten Art ist durch DE 10 2009 060 627 A1 bekannt. Bei dieser Elektrodenanordnung ist das Dielektrikum durch ein flexibles flächiges Material gebildet, das auf seiner zur zu behandelnden Oberfläche zeigenden Seite mit einer Struktur versehen ist, um Luftführungsbereiche auszubilden, wenn das Dielektrikum auf der zu behandelnden Oberfläche aufliegt. Die flächige Elektrode ist flexibel ausgebildet und am Dielektrikum so befestigt, dass eine Schicht des Dielektrikums die Elektrode von der zu behandelnden Oberfläche abschirmt. Insbesondere kann die Elektrode vollständig von dem Material des Dielektrikums umschlossen sein, wobei lediglich ein Hochspannungsanschluss aus dem Dielektrikum herausgeführt ist. Die bekannte Elektrodenanordnung eignet sich insbesondere für die Behandlung der Hautoberfläche eines menschlichen oder tierischen Körpers zur Durchführung einer therapeutischen oder insbesondere kosmetischen Behandlung. Durch die dadurch verbesserte Aufnahmefähigkeit der Haut für kosmetische Wirkstoffe sind in effizienter Weise kosmetische Behandlungen, wie Faltenglättungen, Porenverkleinerungen usw. möglich. Darüber hinaus kann eine bakterizide und fungizide Wirkung der Plasmabehandlung auch für die Behandlung von gesunden oder verwundeten Hautflächen ausgenutzt werden. Die flexible Ausbildung der bekannten Elektrodenanordnung ermöglicht eine Anpassung an die unregelmäßig geformte Oberfläche. Sofern elastische Rückstellkräfte dabei nicht zu groß sind, behält die Elektrodenanordnung ihre Verformung bei. Dabei ist allerdings die Anpassbarkeit an kleine lokale Unregelmäßigkeiten der Oberfläche begrenzt.

US 6,413,255 B1 beschreibt ein Gerät zur Behandlung der Haut eines menschlichen Körpers, bei der über das Gerät Wärme in die unterhalb der Epidermis liegende Dermis der menschlichen Haut eingetragen werden soll. Hierzu ist eine kapazitive Einbringung der Wärme durch eine Hochfrequenzanregung einer Elektrode vorgesehen, die zur Haut hin mit einem Dielektrikum abgedeckt sein kann, das als verformbares Material eine Anpassung an die Oberfläche der Haut bewirken kann. Die Elektrodenanordnung kann dabei einen membranähnlichen Abschnitt aus einer leitenden Elektrodenschicht und einem flexiblen Dielektrikum bilden, der insbesondere bei der Zuführung eines Kältemittels zum anschließenden Abkühlen der Haut dadurch gedehnt wird, dass das Kältemittel verdunstet und in der Elektrodenanordnung für einen Überdruck sorgt, der den membranähnlichen Abschnitt der Elektrode zu einem Ausbeulen - und damit zu einer verstärkten Anlage an der Haut - führt. Diese Druckschrift offenbart keine Elektrodenanordnung zur Ausbildung eines dielektrisch behinderten Plasmas zwischen der Elektrodenanordnung und der Hautoberfläche.

Der vorliegenden Erfindung liegt die Aufgabe zugrunde, eine Elektrodenanordnung der eingangs erwähnten Art hinsichtlich der Anpassbarkeit an unregelmäßig geformte Oberflächen zu verbessern.

Zur Lösung dieser Aufgabe ist eine Elektrodenanordnung der eingangs erwähnten Art in einer ersten Ausführungsform der Erfindung gekennzeichnet durch ein flächenelastisches Andruckmittel zum Andruck auf der der Oberfläche abgewandten Rückseite des Elektrodenelements derart, dass das Elektrodenelement unter lokaler Verformung an Unregelmäßigkeiten der Oberfläche selbsttätig anpassbar ist, wobei das flächenelastische Andruckmittel ein elastisches Material ist, das in einem als Stützlager dienenden Gehäuseteil befestigt ist.

Zur Lösung der Aufgabe ist ferner eine Elektrodenanordnung der eingangs erwähnten Art in einer zweiten Ausführungsform gekennzeichnet durch ein flächenelastisches Andruckmittel zum Andruck auf der der Oberfläche abgewandten Rückseite des Elektrodenelements derart, dass das Elektrodenelement unter lokaler Verformung an Unregelmäßigkeiten der Oberfläche selbsttätig anpassbar ist, wobei das flächenelastische Andruckmittel aus einer Vielzahl von Federelementen gebildet ist, die sich einseitig an einem als Stützlager dienenden Gehäuseteil abstützen und mit Anlageflächen ein Andruckarray auf der Rückseite des Elektrodenelements bilden.

Die erfindungsgemäße Elektrodenanordnung sorgt somit selbsttätig für die Anpassung des Elektrodenelements an Unregelmäßigkeiten der Oberfläche, auch wenn die Unregelmäßigkeiten nur eine geringe flächige Ausdehnung aufweisen. Durch die erfindungsgemäße Anordnung eines flächenelastischen Andruckmittels auf der Rückseite der Elektrodenanordnung bleibt ein gleichmäßiger Andruck des Elektrodenelements auf der zu behandelnden Oberfläche gewährleistet. Das "flächenelastische" Andruckmittel bewirkt, dass das flächige Elektrodenelement gleichmäßig in Richtung der zu behandelnden Oberfläche gedrückt wird, und zwar über die gesamte Andruckfläche mit einer im Wesentlichen gleichen Andruckkraft.

Die Andruckkraft des flächenelastischen Andruckmittels kann gemäß der ersten Ausführungsform durch ein elastisches Material generiert werden, das in einem als Stützlager dienenden Gehäuseteil befestigt ist. Das Gehäuseteil kann starr, aber auch mit einer gewissen Flexibilität ausgebildet sein, wobei das Gehäuse steifer sein muss als das elastische Material, um als Stützlager dienen zu können. Das elastische Material, das vorzugsweise ein weichelastisches Material ist, kann dabei bei der Anlage der Elektrodenanordnung auf der zu behandelnden Oberfläche komprimiert werden, sodass die Rückstellkraft des elastischen Materials die flächig wirkende Andruckkraft für die Elektrodenanordnung bewirkt. Weist die Oberfläche unregelmäßige Verformungen auf, sorgt die Andruckkraft des elastischen Materials dafür, dass sich die flexibel ausgestaltete Elektrodenanordnung dieser Verformung anpassen kann. Auf diese Weise gelingt die Anpassung an Unregelmäßigkeiten, deren Ausdehnung in der Fläche im Zentimeterbereich bis hinunter in den Bereich einiger Millimeter beträgt. Das die Andruckkraft bewirkende Material kann dabei normales weichelastisches Material sein, beispielsweise durch einen offenzelligen oder geschlossenzelligen Schaumkunststoff gebildet sein. Das elastische Material kann auch ein Elastomermaterial sein. Darüber hinaus ist es möglich, eine senkrecht zur Andruckrichtung stehende Oberfläche des Materials profiliert auszubilden, um so die elastische Andruckkraft in Abhängigkeit vom Verformungsweg zu steuern. So ist es beispielsweise möglich, das elastische Material auf der von der Elektrodenanordnung abgewandten Seite mit konisch oder abgerundet ausgebildeten Vorsprüngen an dem Gehäuseteil anliegen zu lassen, sodass sich aufgrund der Verformung der Vorsprünge eine progressiv ansteigende Rückstellkraft ergibt. Die Erzeugung der Andruckkraft mit einem komprimierbaren elastischen Material ist konstruktiv einfach zu realisieren.

Gemäß der zweiten Ausführungsform der Erfindung besteht das flächenelastische Andruckmittel aus einer Vielzahl von Federelementen, die sich einseitig an dem Gehäuseteil abstützen und mit Anlageflächen ein Andruckarray auf der Rückseite der Elektrodeneinrichtung bilden. Hierdurch lässt sich eine Andruckeinrichtung nach Art einer Federkernmatratze ausbilden.

Das Gehäuseteil, das für den Andruck der Elektrodenanordnung an die Oberfläche als Widerlager für das flächenelastische Andruckmittel dient, hat vorzugsweise eine flächige Ausdehnung, mit der es die flächige Ausdehnung der Elektrodeneinrichtung überragt, vorzugsweise allseitig überragt.

Das flächenelastische Andruckmittel kann aus einem dielektrischen Material gebildet sein, das die Rückseite der Elektrodenanordnung abdeckt. Dadurch ist es möglich, dass die Elektrode zur zu behandelnden Oberfläche abdeckende Dielektrikum dünner und noch flexibler auszubilden, weil die Isolierung der die Hochspannung führende Elektrode auf der Rückseite zumindest auch durch das flächenelastische Andruckmaterial erfolgen kann. Selbst wenn die Elektrode in ein Dielektrikum vollständig eingebettet, beispielsweise eingegossen, wird, um so eine Berührungssicherheit zu gewährleisten, kann das Andruckmittel zu einer zusätzlichen Sicherheit beitragen.

Auch bei der erfindungsgemäßen Elektrodenanordnung ist es bevorzugt, dass die Wirkfläche des Dielektrikums eine Struktur aufweist, die Luftzwischenräume ausbildet, in denen das Plasma entstehen kann, wenn die Wirkfläche an der zu behandelnden Oberfläche anliegt. Bevorzugt ist dabei, dass die Struktur Noppen aufweist, deren Stirnflächen zur Anlage an der zu behandelnden Oberfläche ausgebildet sind, wie dies grundsätzlich durch DE 10 2009 060 627 A1 bekannt ist.

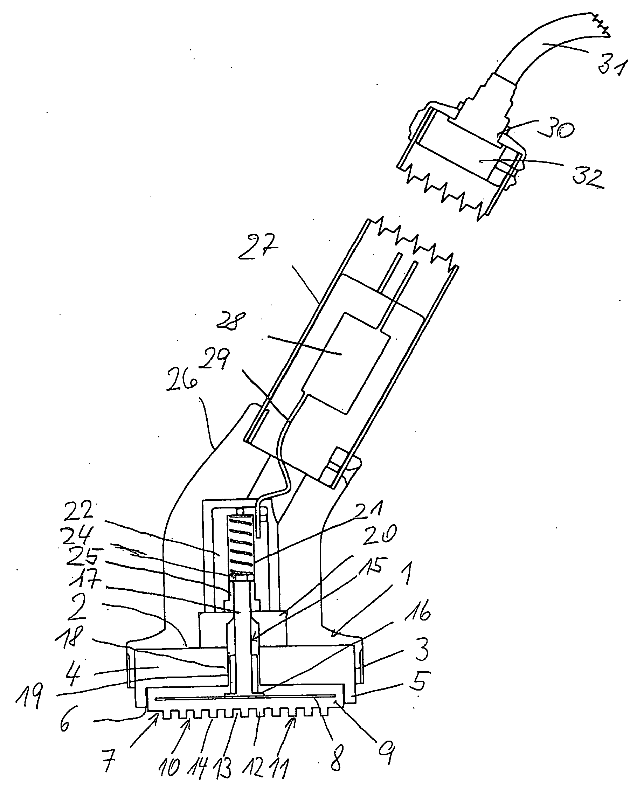

Die Erfindung soll im Folgenden anhand eines in der Zeichnung dargestellten Ausführungsbeispiels näher erläutert werden.

Die einzige Zeichnungsfigur zeigt ein Gehäuseteil 1, das topfförmig mit einer flächigen Bodenwand 2 und einer zylindrischen umlaufenden Mantelwand 3 ausgebildet ist. Das Gehäuseteil 1 kann dabei im Querschnitt rund, rechteckig oder mehreckig ausgebildet sein. Das Gehäuseteil 1 ist auf der der Bodenwand 2 gegenüberliegenden Seite offen ausgebildet und dient somit als Aufnahme für ein flächenelastisches Andruckmittel 4 in Form eines einstückig elastischen Materialstücks, beispielsweise aus einem vorzugsweise weichelastischen Schaumkunststoff.

Das elastische Andruckmittel 4 ist auf der von der Bodenwand 2 abgewandten Fläche mit einer großflächigen Ausnehmung versehen, die einen durch einen umlaufenden Rand 5 begrenzten Aufnahmeraum 6 ausbildet. In den einseitig offenen Aufnahmeraum 6 ist ein Elektrodenelement 7 passig eingesetzt, das aus einer flächigen Elektrode 8 besteht, die allseitig von einem flächigen und flexiblen Dielektrikum 9 umgeben ist. Das Dielektrikum 9 ragt mit einer Wirkfläche 10 über den Rand 5 des Andruckmittels 4 hinaus. Die Wirkfläche 10 ist mit einer Struktur 11 versehen, mit der sie an einer zu behandelnden (nicht dargestellten) Oberfläche anliegen kann. Die Struktur 11 besteht aus Vorsprüngen 12, zwischen denen sich Zwischenräume 13 befinden. Die Zwischenräume 13 sind beispielsweise luftgefüllt und bilden die Räume, in denen sich das Plasma ausbilden kann, wenn die Wirkfläche 10 über die Struktur 11 an der zu behandelnden Oberfläche anliegt.

In der dargestellten bevorzugten Ausführungsform sind die Vorsprünge 12 durch Noppen gebildet, die miteinander fluchtende ebene Stirnflächen 14 aufweisen, die in ihrer Gesamtheit eine Anlagefläche für die zu behandelnde Oberfläche, beispielsweise Hautoberfläche, bilden.

Die Elektrode 8 und das Dielektrikum 9 sind aus einem leicht verformbaren Material gebildet und können sich daher Unebenheiten der zu behandelnde Oberfläche aufgrund des durch das Andruckmittel 4 generierten Andrucks anpassen, sodass eine gleichmäßige Plasmaausbildung in den Zwischenräumen 13 auch bei unregelmäßig geformten Oberflächen gewährleistet ist.

Die Elektrode 8 ist mit einem Hochspannungsanschluss 15 kontaktiert, der mit einem flächigen Endstück 16 an der Elektrode anliegt, das einstückig in einen bolzenartigen Körper 17 übergeht, mit dem der Hochspannungsanschluss 5 durch eine Durchgangsöffnung 18 des elastischen Andruckmittels 4 hindurchgeführt ist. Eine unmittelbare Anlage des bolzenartigen Körpers 17 an dem Andruckmittel 4 wird durch ein isolierendes Hülsenstück 19 vermieden, das den bolzenartigen Körper 17 innerhalb der Durchgangsöffnung 18 des Andruckmittels 4 umgibt und sich bis zum flächigen Endstück 16 des Hochspannungsanschluss 15 erstreckt. Fluchtend mit der Bodenwand 2 des Gehäuseteils 1 ist in das Gehäuseteil 1 ein Isolierkörper 20 eingesetzt, durch den der bolzenartige Körper 17 hindurchragt und sich bis in einen Innenraum 21 eines nach unten offenen metallischen Hochspannungsblocks 22 erstreckt. Der bolzenartige Körper 17 wird über eine Druckfeder 23 an einer gegenüberliegenden Stirnwand des Innenraums 21 abgestützt. Hierzu ist der bolzenartige Körper 17 mit einem stirnseitigen Abschlussstück 24 versehen, dessen Außendurchmesser dem Innendurchmesser des Innenraums 21 entspricht. Zwischen dem Abschlussstück 24 und dem isolierstück 20 befindet sich ein elektrisch leitendes, flexibles Übergangsstück 25, das mit einer nach innen gerichteten Vorspannung an den bolzenartigen Körper 17 anliegt und so eine elektrische Verbindung zwischen dem Hochspannungsblock 22 und dem bolzenartigen Körper 17 des Hochspannungsanschlusses 15 bewirkt. Das Übergangsstück 25 kann dabei nach Art eines metallischen Faltenbalges oder mit nach innen vorstehenden und durch den bolzenartigen Körper 17 nach radial außen gebogenen Lamellen versehen sein.

Das Gehäuseteil 1 weist an einem abgewinkelten Endstück 26 eine Aufnahme für ein rohrförmiges Griffteil 27 auf. In dem rohrförmigen Griffteil befindet sich - schematisch dargestellt - ein Hochspannungsgenerator 28, der auf einer Ausgangsleitung 29 ein Hochspannungspotential abgibt. Die Ausgangsleitung 29 ist mit dem Hochspannungsblock 22 in einer herkömmlichen Weise verbunden, sodass der gesamte Hochspannungsblock auf dem Hochspannungspotential liegt, das über das Übergangsstück 25 auf den Hochspannungsanschluss 15 und von dort auf die Elektrode 8 übertragen wird.

Das rohrförmige Griffteil ist an seinem oberen Ende mit einer Kabeldurchführung 30 für ein Anschlusskabel 31 abgeschlossen. Das Anschlusskabel 31 transportiert eine Niedervoltspannung von beispielsweise 6 V, die in einen Spannungswandler 32 innerhalb des Griffteils 27 auf ca. 400 V gewandelt wird. Diese Ausgangsspannung bildet die Eingangsspannung für den Hochspannungsgenerator 28, der eine pulsförmige Hochspannung generiert, indem beispielsweise höherfrequente Zündfunken generiert werden, deren Stromfluss in einem Transformator zu Hochspannungsspitzen hochtransformiert wird. Selbstverständlich sind auch alle anderen vorbekannten Hochspannungsgeneratoren einsetzbar.

Das dargestellte Ausführungsbeispiel stellt somit ein Handgerät dar, das an dem - selbstverständlich isolierenden - Griffteil 27 ergriffen werden kann. Die Stromzuführung zu dem Griffteil 27 ist eine ungefährliche Niedervolt-Stromleitung. Die Hochspannungserzeugung, die für die Plasmaausbildung benötigt wird, findet im dem Griffstück selbst statt. Die generierte Hochspannung gelangt auf den Hochspannungsblock 22, der in dem isolierenden Gehäuseteil 1 sicher befestigt ist und über den das Hochspannungspotential auf den Hochspannungsanschluss 15 und damit auf die Elektrode 8 gelangt. Der Hochspannungsgenerator 28 erzeugt das Hochspannungspotential, sodass beispielsweise für die Behandlung einer Hautoberfläche diese als Gegenelektrode für das erzeugte Plasma fungieren kann.

## Patentansprüche

1. Elektrodenanordnung zur Ausbildung eines dielektrisch behinderten Plasmas zwischen einer Wirkfläche (10) der Elektrodenanordnung und einer als Gegenelektrode fungierenden Oberfläche, mit einer flexiblen, flächigen Elektrode (8), die mit einer Hochspannungsquelle (28) verbindbar ist und mit einem flächigen, flexiblen und die Wirkfläche (10) bildenden Dielektrikum (9), das mit der flächigen Elektrode (8) zu einem Elektrodenelement (7) verbunden ist und die Elektrode (8) zur zu behandelnden Oberfläche hin vollständig abdeckt, **gekennzeichnet durch** ein flächenelastisches Andruckmittel (4) zum Andruck auf der der Oberfläche abgewandten Rückseite des Elektrodenelements (7) derart, dass das Elektrodenelement (7) unter lokaler Verformung an Unregelmäßigkeiten der Oberfläche selbsttätig anpassbar ist, wobei das flächenelastische Andruckmittel (4) ein elastisches Material ist, das in einem als Stützlager dienenden Gehäuseteil (1) befestigt ist.

2. Elektrodenanordnung zur Ausbildung eines dielektrisch behinderten Plasmas zwischen einer Wirkfläche (10) der Elektrodenanordnung und einer als Gegenelektrode fungierenden Ober-fläche, mit einer flexiblen, flächigen Elektrode (8), die mit einer Hochspannungsquelle (28) verbindbar ist und mit einem flächigen, flexiblen und die Wirkfläche (10) bildenden Dielektrikum (9), das mit der flächigen Elektrode (8) zu einem Elektrodenelement (7) verbunden ist und die Elektrode (8) zur zu behandelnden Oberfläche hin vollständig abdeckt, **gekennzeichnet durch** ein flächenelastisches Andruckmittel (4) zum Andruck auf der der Oberfläche abgewandten Rückseite des Elektrodenelements (7) derart, dass das Elektrodenelement (7) unter lokaler Verformung an Unregelmäßigkeiten der Oberfläche selbsttätig anpassbar ist, wobei das flächenelastische Andruckmittel (4) aus einer Vielzahl von Federelementen gebildet ist, die sich einseitig an einem als Stützlager dienenden Gehäuseteil (1) abstützen und mit Anlageflächen ein Andruckarray auf der Rückseite des Elektrodenelements (7) bilden.

3. Elektrodenanordnung nach Anspruch 1 oder 2, **dadurch gekennzeichnet, dass** das Andruckmittel (4) die Rückseite des Elektrodenelements (7) mit einem dielektrischen Material abdeckt.

4. Elektrodenanordnung nach einem der Ansprüche 1 bis 3, **dadurch gekennzeichnet, dass** das Gehäuseteil (1) eine flächige Ausdehnung aufweist, mit der es die flächige Ausdehnung des Elektrodenelements (7) überragt.

5. Elektrodenanordnung nach Anspruch 4, **dadurch gekennzeichnet, dass** die flächige Ausdehnung des Gehäuseteils (1) die flächige Ausdehnung des Elektrodenelements (7) allseitig überragt.

6. Elektrodenanordnung nach einem der Ansprüche 1 bis 5, **dadurch gekennzeichnet, dass** das Gehäuseteil (1) eine elektrische Durchführung für einen Hochspannungsanschluss (15) aufweist.

7. Elektrodenanordnung nach einem der Ansprüche 1 bis 6, **dadurch gekennzeichnet, dass** die Wirkfläche (10) des Dielektrikums (9) eine Struktur (11) aufweist, die Zwischenräume (13) ausbildet, in denen das Plasma ausbildbar ist, wenn die Wirkfläche (10) über die Struktur (11) an der Oberfläche anliegt.

8. Elektrodenanordnung nach Anspruch 7, **dadurch gekennzeichnet, dass** die Struktur (11) Noppen aufweist, deren Stirnflächen (14) zur Anlage an der Oberfläche ausgebildet sind.

## Claims

1. Electrode arrangement for forming a dielectrically impeded plasma between an active surface (10) of the electrode arrangement and a surface which acts as a counterelectrode, having a flexible, flat electrode (8) which can be connected to a high-voltage source (28), and having a flat, flexible dielectric (9) which forms the active surface (10) and is connected to the flat electrode (8) to form an electrode element (7) and completely covers the electrode (8) with respect to the surface which is to be treated, **characterized by** an area-elastic contact-pressure means (4) for pressing on the rear face of the electrode element (7), which rear face is averted from the surface, in such a way that the electrode element (7) can be automatically adapted to irregularities of the surface by local deformation, wherein the area-elastic contact-pressure means (4) is an elastic material which is fastened in a housing part (1) which serves as a supporting bearing.

2. Electrode arrangement for forming a dielectrically impeded plasma between an active surface (10) of the electrode arrangement and a surface which acts as a counterelectrode, having a flexible, flat electrode (8) which can be connected to a high-voltage source (28), and having a flat, flexible dielectric (9) which forms the active surface (10) and is connected to the flat electrode (8) to form an electrode element (7) and completely covers the electrode (8) with respect to the surface which is to be treated, **characterized by** an area-elastic contact-pressure means (4) for pressing on the rear face of the electrode element (7), which rear face is averted from the surface, in such a way that the electrode element (7) can be automatically adapted to irregularities of the surface by local deformation, wherein the area-elastic contact-pressure means (4) is formed from a large number of spring elements which are supported at one end against a housing part (1) which serves as a supporting bearing, and, with contact surfaces, form a contact-pressure array on the rear face of the electrode element (7).

3. Electrode arrangement according to Claim 1 or 2, **characterized in that** the contact-pressure means (4) covers the rear face of the electrode element (7) with a dielectric material.

4. Electrode arrangement according to one of Claims 1 to 3, **characterized in that** the housing part (1) has a flat extent with which it projects beyond the flat extent of the electrode element (7).

5. Electrode arrangement according to Claim 4, **characterized in that** the flat extent of the housing part (1) projects beyond the flat extent of the electrode element (7) on all sides.

6. Electrode arrangement according to one of Claims 1 to 5, **characterized in that** the housing part (1) has an electrical bushing for a high-voltage connection (15).

7. Electrode arrangement according to one of Claims 1 to 6, **characterized in that** the active surface (10) of the dielectric (9) has a structure (11) which forms intermediate spaces (13) in which the plasma can form when the active surface (10) bears against the surface by way of the structure (11).

8. Electrode arrangement according to Claim 7, **characterized in that** the structure (11) has studs, the end faces (14) of said studs being designed to bear against the surface.

## Revendications

1. Agencement d'électrode pour la réalisation d'un plasma à barrière diélectrique entre une surface active (10) de l'agencement d'électrode et une surface faisant office d'électrode antagoniste, comprenant une électrode flexible surfacique (8), qui peut être reliée à une source à haute tension (28), et avec un diélectrique surfacique flexible (9) formant la surface active (10), qui est relié à l'électrode surfacique (8) pour donner un élément d'électrode (7) et qui recouvre entièrement l'électrode (8) en direction de la surface à traiter, **caractérisé par** un organe presseur (4) à élasticité surfacique, pour presser sur la face postérieure, détournée de la surface, de l'élément d'électrode (7), de telle façon que l'élément d'électrode (7) s'ajuste automatiquement sous déformation locale aux irrégularités de la surface, et l'organe presseur (4) à élasticité surfacique est un matériau élastique, qui est fixé dans une partie de boîtier (1) formant palier de soutien.

2. Agencement d'électrode pour la réalisation d'un plasma à barrière diélectrique entre une surface active (10) de l'agencement d'électrode et une surface faisant office d'électrode antagoniste, comprenant une électrode flexible surfacique (8), qui peut être reliée à une source à haute tension (28), et avec un diélectrique surfacique flexible (9) formant la surface active (10), qui est relié à l'électrode surfacique (8) pour donner un élément d'électrode (7) et qui recouvre entièrement l'électrode (8) en direction de la surface à traiter, **caractérisé par** un organe presseur (4) à élasticité surfacique, pour presser sur la face postérieure, détournée de la surface, de l'élément d'électrode (7), de telle façon que l'élément d'électrode (7) s'ajuste automatiquement sous déformation locale aux irrégularités de la surface, et l'organe presseur (4) à élasticité surfacique est formé d'une pluralité d'éléments à ressort, qui s'appuient d'un côté sur une partie de boîtier (1) formant palier de soutien et forment, avec des surfaces d'appui, un réseau presseur sur la face postérieure de l'élément d'électrode (7).

3. Agencement d'électrode selon la revendication 1 ou 2, **caractérisé en ce que** l'organe presseur (4) recouvre la face postérieure de l'élément d'électrode (7) avec un matériau diélectrique.

4. Agencement d'électrode selon l'une des revendications 1 à 3, **caractérisé en ce que** la partie de boîtier (1) présente une extension en surface avec laquelle elle dépasse l'extension en surface de l'élément d'électrode (7).

5. Agencement d'électrode selon la revendication 4, **caractérisé en ce que** l'extension en surface de la partie de boîtier (1) dépasse l'extension en surface de l'élément d'électrode (7) sur tous les côtés.

6. Agencement d'électrode selon l'une des revendications 1 à 5, **caractérisé en ce que** la partie de boîtier (1) comporte une traversée électrique pour un raccordement à haute tension (15).

7. Agencement d'électrode selon l'une des revendications 1 à 6, **caractérisé en ce que** la surface active (10) du diélectrique (9) comporte une structure (11) qui forme des intervalles (13) dans lesquels le plasma est capable de se former quand la surface active (10) est appliquée sur la surface au moyen de la structure (11).

8. Agencement d'électrode selon l'une revendication 7, **caractérisé en ce que** la structure (11) comporte des boutons dont les surfaces frontales (14) sont réalisées pour venir en contact contre la surface.
